# EUROPÄISCHE PATENTSCHRIFT

(11) **EP 2 922 819 B1**
(45) Veröffentlichungstag und Bekanntmachung des Hinweises auf die Patenterteilung: **20.04.2016**
(21) Anmeldenummer: 13792876.8
(22) Anmeldetag: 12.11.2013
(51) Int. Cl.: C07D 207/267

(54) **VERFAHREN ZUR REINIGUNG VON N-ALKYL-PYRROLIDONEN**
METHOD FOR PURIFYING N-ALKYLPYRROLIDONES
PROCÉDÉ DE PURIFICATION DE N-ALKYLPYRROLIDONES

(30) Priorität: 22.11.2012 EP 12193748
(43) Veröffentlichungstag der Anmeldung: 30.09.2015
(73) Patentinhaber: BASF SE, 67056 Ludwigshafen am Rhein (DE)
(72) Erfinder: PINKOS, Rolf, 67098 Bad Dürkheim (DE); VOGLER, Thomas, 67071 Ludwigshafen (DE); OTT, Karl, 68723 Plankstadt (DE)
(74) Vertreter: BASF IP Association
(86) Internationale Anmeldenummer: PCT/EP2013/073533
(87) Internationale Veröffentlichungsnummer: WO 2014/079720

(56) Entgegenhaltungen:
- WO-A1-2010/057917
- JP-A- 2007 099 690

## Beschreibung

Die vorliegende Erfindung betrifft ein Verfahren zur Reinigung von N-Alkyl-pyrrolidonen, welche durch eine bereits erfolgte Verwendung mindestens eine der Verunreinigungen der Formel I oder II 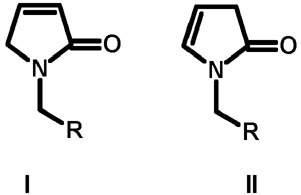 worin R für Wasserstoff oder eine C1- bis C20-Alkylgruppe steht, enthalten.

Dieses Verfahren ist dadurch gekennzeichnet, dass
- dem zu reinigenden N-Alkyl-pyrrolidon eine basische Verbindung zugesetzt wird und die Temperatur des Gemisches spätestens 20 Minuten nach Zusatz der basischen Verbindung mindestens 80°C beträgt und
- das N-Alkyl-pyrrolidon aus dem erhaltenen Gemisch abdestilliert wird.

Weiterhin betrifft die vorliegende Erfindung ein Verfahren zur Verwendung, Reinigung und Wiederverwendung von N-Alkyl-pyrrolidonen, welches dadurch gekennzeichnet ist, dass
a) ein Polymer in einem N- Alkyl-pyrrolidon gelöst wird,
b) Elektroden von Lithium-ionen-akkumulatoren mit diesem Polymer hergestellt werden und dabei ein verunreinigtes N-Alkyl-pyrrolidon zurückgewonnen wird, welches mindestens eine der Verunreinigungen der Formel I oder II 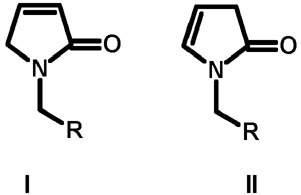 worin R in Formel I und II für Wasserstoff oder eine C1- bis C20-Alkylgruppe steht, enthält,
c) dem zurückgewonnenen N-Alkyl-pyrrolidon eine basische Verbindung zugesetzt wird und die Temperatur des Gemisches spätestens 20 Minuten nach Zusatz der basischen Verbindung auf mindestens 80°C erhöht wird,
d) das N-Alkyl-pyrrolidon aus dem erhaltenen Gemisch abdestilliert wird, und
e) erneut als Lösemittel für die Herstellung von Elektroden für Lithium-ionen-Akkumulatoren verwendet wird.

Zur Herstellung von Lithium-Ionen-Akkumulatoren werden häufig Polymere verwendet, z.B. als polymerer Elektrolyt oder zur Herstellung von Elektroden. Üblich ist z. B. die Verwendung von Polyvinylidenfluorid zur Herstellung der Elektroden.

Dazu wird Polyvinylidenfluorid üblicherweise in einem organischen Lösemittel gelöst und durch Zusatz von Lithiumspeichermaterialien und gegebenenfalls leitenden Zusatzstoffen eine Suspension hergestellt. Durch Beschichtung eines Trägers, zum Beispiel eines metallischen Trägers mit dieser Suspension und anschließende Entfernung des Lösemittels wird die Elektrode erhalten. Als geeignete Lösemittel haben sich N-Alkyl-pyrrolidone, insbesondere N-Methyl-pyrrolidon erwiesen.

Durch die vorstehende Verwendung kann das Lösemittel verunreinigt werden. Durch den Beschichtungsprozess werden Verunreinigungen eingebracht, auch entstehen aufgrund der erforderlichen hohen Temperaturen und/oder Anwesenheit oxidierend wirkender Verbindungen Nebenprodukte des verwendeten Lösemittels. Bei N-Methyl-pyrrolidon (NMP) entstehen durch unerwünschte Nebenreaktionen Verbindungen der obigen Formel I und II, worin R für ein H-Atom steht. Diese haben einen ähnlichen Siedepunkt wie NMP und sind daher destillativ kaum von NMP abzutrennen.

Für die Herstellung von langlebigen Lithium-Ionen Akkumulatoren dürfen nur Lösemittel mit hoher Reinheit und Wasserfreiheit verwendet werden. Einmal verwendetes Lösemittel kann nur erneut eingesetzt werden, wenn es gereinigt wurde und die hohen Anforderungen an die Reinheit und Wasserfreiheit wieder erfüllt.

Aus JP 11071346 und JP 10310795 ist bekannt, NMP, welches als Lösemittel für Polyvinylidenfluorid verwendet wird, durch Zugabe von sauren Verbindungen, insbesondere durch Kontakt mit sauren Feststoffen, zu reinigen.

WO 2010/057917 beschreibt die Reinigung von NMP, welches als Lösemittel für Polyvinylidenfluorid und die Herstellung von Lithium-Ionen-Akkumulatoren verwendet wurde. Die Reinigung erfolgt durch Zugabe von Aktivkohle, es handelt sich daher um eine Reinigung durch Adsorption.

Auch der Zusatz von Alkali- oder Erdalkalimetallhydroxiden zu NMP ist bereits beschrieben. So ist aus JP 2004284958 und JP 2007099690 bekannt, NMP in Gegenwart von Alkali- oder Erdalkalihydroxiden zu destillieren, um so die Bildung von Peroxiden zu verhindern und das NMP zu stabilisieren.

In US 4965370, JP 2001354769 und JP 11349566 wird die Reinigung von NMP, welches bei der Polyarylensulfid-Herstellung verwendet wurde, beschrieben; durch Zusatz von Alkali- oder Erdalkalihydroxiden können jeweils Verunreinigungen aus dem Polyarylensulfid-Prozess entfernt und das NMP für diesen Prozess erneut verwendet werden.

JP 03014559 offenbart die Entfernung von N-Methylsuccinimid und Phenol aus NMP durch Zusatz von Metallhydroxiden oder Metallcarbonaten. Auch hier handelt es sich um NMP aus dem Polyarylensulfid-Prozess.

In WO 03/053924 wird ein Verfahren zur Herstellung von NMP aus gamma-Butyrolacton und Methylamin beschrieben. Hier werden Metallhydroxide eingesetzt, um überschüssiges gamma-Butyrolacton durch Salzbildung zu entfernen.

Aufgabe der vorliegenden Erfindung, war ein Verfahren zur Reinigung von bereits verwendetem N-Alkyl-pyrrolidon, insbesondere NMP, welches die Wiederverwendung der N-Alkyl-pyrrolidone für beliebige Anwendungen, insbesondere für die Herstellung von Lithium-Ionen-akkumulatoren erlaubt. Das Verfahren soll möglichst einfach und effektiv sein. Durch das Verfahren sollen eingebrachte oder entstandene Verunreinigungen der obigen Formeln I und II möglichst vollständig entfernt werden.

Demgemäß wurde das oben definierte Verfahren gefunden.

Das erfindungsgemäße Verfahren ist ein Verfahren zur Reinigung von N-Alkyl-pyrrolidonen, welche bereits mindestens einmal verwendet wurden. Zum Beispiel können die N-Alkyl-pyrrolidone als Lösemittel verwendet worden sein.

Bevorzugt handelt es sich um N-Alkyl-pyrrolidone der Formel A 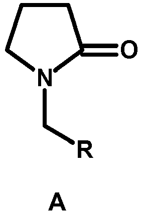 wobei R für Wasserstoff oder eine C1- bis C20-Alkylgruppe steht.

Insbesondere steht R in Formel A für Wasserstoff oder eine C1- bis C3- Alkylgruppe, besonders bevorzugt steht R für Wasserstoff oder eine Methylgruppe. Steht R für Wasserstoff handelt es sich um N-Methyl-pyrrolidon (NMP), steht R für eine Methylgruppe, handelt es sich um N-Ethylpyrrolidon (NEP). Besonders bevorzugt handelt es sich um NMP oder NEP. Ganz besonders bevorzugt handelt es sich um NMP.

Durch die bereits erfolgte Verwendung enthält das N-Alkylpyrrolidon mindestens eine der Verunreinigungen der Formel I oder II 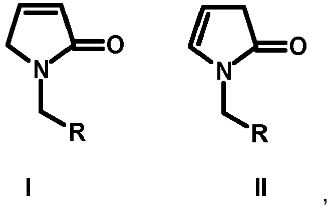 worin R für Wasserstoff oder eine C1- bis C20-Alkylgruppe steht.

Die Verbindungen der Formel I und II sind Isomere, welche häufig im Gemisch auftreten. Das zu reinigende N-Alkyl-pyrrolidon kann daher beide Verbindungen enthalten. Bei R handelt es sich vorzugsweise um Wasserstoff oder eine C1- bis C4-Alkylgruppe.

Wenn es sich bei dem zu reinigenden N-Alkyl-pyrrolidon um NMP handelt, steht R in Formeln I und II für Wasserstoff.

Wenn es sich bei dem zu reinigenden N-Alkyl-pyrrolidon um NEP handelt, steht R in Formeln I und II für eine Methylgruppe.

Der Gehalt an Verbindungen der Formeln I und II im N-Alkyl-pyrrolidon kann insbesondere 0,0005 bis 1 Gewichtsteil auf 100 Gewichtsteile N-Alkyl-pyrrolidon betragen. Bei dem vorliegenden Verfahren wird insbesondere ein N-Alkyl-pyrrolidon verwendet, das einen Gehalt an Verbindungen der Formeln I und II von insgesamt 0,0005 bis 0,5 besonders bevorzugt von 0,0005 bis 0,1 Gewichtsteilen auf 100 Gewichtsteile N-Alkyl-pyrrolidon hat.

Neben den Verbindungen der Formeln I und II kann das zu reinigende N-Alkyl-pyrrolidon weitere organische Verbindungen als Verunreinigungen enthalten

Weitere organische Verbindungen, die als Verunreinigung enthalten sein können, sind zum Beispiel solche der nachstehenden Formeln 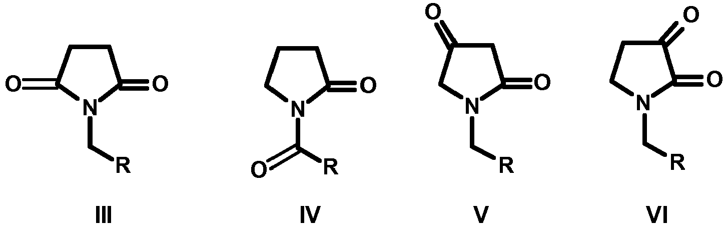 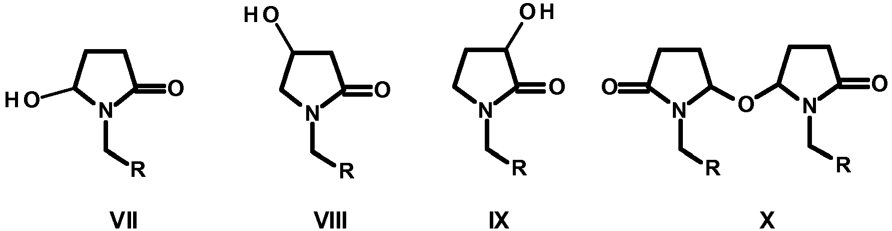 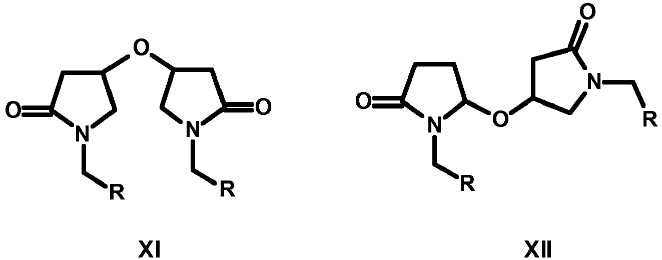 wobei R jeweils für Wasserstoff oder eine C1 - bis C20 Alkylgruppe steht.

Neben den Verbindungen der Formeln I, II und gegebenenfalls III bis XII kann das zu reinigende N-Alkyl-pyrrolidon weitere organische Verbindungen enthalten, die bereits aus dem Herstellungsprozess des N-Alkyl-pyrrolidons stammen und nicht zu den Verunreinigungen gezählt werden. Neben gamma-Butyrolacton und dem zur Herstellung des N-Alkyl-pyrrolidons verwendeten Amin (Methylamin im Falle des NMP) sind diese weiteren organischen Verbindungen Derivate des N-Alkyl-pyrrolidons, die an den Ringkohlenstoffatomen mit Alkylresten substituiert sind. Bevorzugte Alkylreste sind Methyl- und Ethylgruppen und können an allen Ringkohlenstoffatomen einzeln oder als Mischung vorliegen. Der Gehalt dieser substituierten N-Alkyl-pyrrolidone beträgt im Allgemeinen weniger als 1 Gewichtsteil, insbesondere weniger als 0,5 Gewichtsteile auf 100 Gewichtsteile N-Alkyl-pyrrolidon. Der Gehalt an gamma-Butyrolacton beträgt im Allgemeinen weniger als 0,1 Gewichtsteile, insbesondere weniger als 0,05 Gewichtsteile auf 100 Gewichtsteile N-Alkyl-pyrrolidon. Der Gehalt an dem zur Herstellung des N-Alkyl-pyrrolidons verwendeten Amin beträgt im Allgemeinen weniger als 0,005 Gewichtsteile, insbesondere weniger als 0,003 Gewichtsteile auf 100 Gewichtsteile N-Alkyl-pyrrolidon.

Das zu reinigende N-Alkyl-pyrrollidon enthält auf 100 Gewichtsteile N-Alkyl-pyrrolidon im Allgemeinen insgesamt 0,0005 bis 5 Gewichtsteile, insbesondere von 0,0005 bis 2 Gewichtsteile organischer Verbindungen der Formeln I bis XI als Verunreinigungen.

Das zu reinigende N-Alkyl-pyrrolidon kann weiterhin Wasser enthalten bzw. im Gemisch mit Wasser vorliegen. Durch vorangegangene Verwendungen können große Mengen Wasser eingebracht worden sein.

Das zu reinigende N-Alkyl-pyrrolidon kann sich daher z.B. wie folgt zusammensetzen aus
100 Gewichtsteilen N-Alkyl-pyrrolidon,
insgesamt 0,0005 bis 5 Gewichtsteile organische Verbindungen der Formeln I bis XII als Verunreinigungen und
0,01 bis 200 Gewichtsteilen Wasser.

Das zu reinigende N-Alkyl-pyrrolidon kann sich insbesondere wie folgt zusammensetzen aus
100 Gewichtsteilen N-Alkyl-pyrrolidon
insgesamt 0,0005 bis 2,5 Gewichtsteile organischen Verbindungen der Formeln I bis XII als Verunreinigungen und 0,1 bis 100 Gewichtsteilen Wasser.

Dem zu reinigenden N-Alkyl-pyrrolidon wird eine basische Verbindung zugesetzt. Unter dem Begriff basische Verbindung werden im Folgenden auch Mischungen von verschiedenen basischen Verbindungen verstanden.

Bei der basischen Verbindung handelt es sich vorzugsweise um eine Verbindung, welche in einer Menge von 5 Gewichtsteilen in 100 Gewichtsteilen Wasser (bei 20°C, 1 bar) einen pH-Wert von mindestens 8, besonders bevorzugt von mindestens 9 bewirkt. Bei der vorstehenden Definition ist es nicht notwendig, dass sich die 5 Gewichtsteile der basischen Verbindung vollständig in Wasser lösen, entscheidend ist nur, dass nach Zugabe der basischen Verbindung die pH-Erhöhung eintritt.

Bei der basischen Verbindung kann es sich um organische oder anorganische Verbindungen handeln. In Betracht kommen zum Beispiel um Alkali-, Erdalkalihydroxide, Alkoholate, Carbonate, Carboxylate, komplexe Hydride, Ammoniak oder Amine. Genannt seien zum Beispiel LiOH, NaOH, KOH, Mg(OH)₂, Ca(OH)₂, Ca(HCO₃)₂, CaCO₃, Natriumoxalat, Natriumborhydrid, Lithiumaluminiumhydrid, Natriummethylat, Natriumethanolat, Kaliummethylat, Kaliumethanolat und dem N-Alkyl-substituierten Pyrrolidon entsprechenden Alkylamin.

Besonders bevorzugt handelt es sich um anorganische Verbindungen, insbesondere um Alkalimetall-haltige Basen oder Erdalkalimetall-haltige Basen.

Ganz besonders bevorzugt handelt es sich bei der zugesetzten basischen Verbindung um NaOH, KOH, Natriummethylat oder Kaliummethylat.

In einer besonders bevorzugten Ausführungsform handelt es sich um Natriumhydroxid.

Die basische Verbindung kann dem zu reinigenden N-Alkyl-pyrrolidon in Substanz oder als Lösung zugesetzt werden. Wird die basische Verbindung in Lösung zugesetzt, so kann es sich bei dem Lösungsmittel zum Beispiel um Wasser oder organische Lösemittel, z. B. auch das jeweilige N-Alkyl-pyrrolidon oder Gemische des jeweiligen N-Alkylpyrrolidon mit Wasser handeln.

Die basische Verbindung wird vorzugsweise in Mengen von 0,01 bis 10 Gewichtsteilen auf 100 Gewichtsteile des zu reinigenden N-Alkyl-pyrrolidon (Summe aus N-Alkylpyrrolidon und aller darin enthaltenen organischen Bestandteile, wie Verunreinigungen und Derivate) eingesetzt. Besonders bevorzugt wird sie in Mengen von 0,01 bis 1 und ganz besonders bevorzugt in Mengen von 0,01 bis 0,5, insbesondere von 0,05 bis 0,5 Gewichtsteile bezogen auf 100 Gewichtsteile des zu reinigenden N-Alkyl-pyrrolidon eingesetzt.

Spätestens 20 Minuten, vorzugsweise spätestens 10 Minuten, besonders bevorzugt spätestens 5 Minuten, ganz besonders bevorzugt spätestens 2 Minuten nach Zusatz der basischen Verbindung beträgt die Temperatur des erhaltenen Gemisches aus zu reinigendem N-Alklyl-pyrrolidon und basischer Verbindung mindestens 80°C.

Vorzugsweise beträgt die Temperatur mindestens 100°C.

Die vorstehende Temperatur kann leicht eingestellt werden, indem
- die basische Verbindung und das zu reinigende N-Alkyl-pyrrolidon zusammengegeben werden und das erhaltene Gemisch schnell aufgeheizt wird, damit in der vorgeschriebenen Zeit die notwendige Temperatur erreicht wird (Alternative 1) oder
- das zu reinigende N-Alkyl-pyrrolidon bereits vorher aufgeheizt wurde, so dass nach der Zugabe der basischen Verbindung die gewünschte Temperatur unmittelbar gegeben ist (Alternative 2).

Eine besondere Ausführungsform der Alternative 2 ist die Zugabe der basischen Verbindung direkt bei der Destillation, durch welche das gereinigte N-Alkyl-pyrrolidon abgetrennt wird.

Ohne die vorstehenden Temperaurerhöhung kann leicht Feststoff ausfallen. Nachteilig dabei ist, dass beispielsweise Rohrleitungen und Ventile verstopfen können, was ein Abstellen der Anlage nötig macht und einen entsprechenden Reinigungsaufwand nach sich zieht.

N-Alkyl-pyrrolidon wird aus dem Gemisch abdestilliert. Das gereinigte N-Alkyl-pyrrolidon wird vorzugsweise am Kopf der Kolonne abgezogen.

Die Destillation wird vorzugsweise bei einer Temperatur von 50 bis 350°C, besonders bevorzugt von 100 bis 250 und einem Druck von 0,3 bis 5 bar durchgeführt.

Als Kolonnen für die Destillation eignen sich dem Fachmann bekannte Kolonnen. Bevorzugt sind Packungskolonnen, Bodenkolonnen mit Siebböden, Kolonnen mit dual-flow-Böden, Kolonnen mit Glockenböden oder mit Ventilböden ausgestattete Rektifikationskolonnen, Trennwandkolonnen oder Dünnschicht- und Fallfilmverdampfer.

Vorzugsweise wird die Destillation in Abwesenheit von Sauerstoff durchgeführt. Unter Abwesenheit wird hier verstanden, dass der Sauerstoffvolumenanteil kleiner als 0,1%, insbesondere kleiner 0,01 und ganz besonders kleiner 0,001 %, bezogen auf das Gesamtvolumen der Destillationskolonne ist.

Vorzugsweise wird bereits die Zugabe der basischen Verbindung zum N-Alkyl-pyrrolidon unter Inertgas-Atmosphäre durchgeführt, so dass während des gesamten Verfahrens der Sauerstoffgehalt vermindert ist. Vorzugsweise wird das gesamte Verfahren in Abwesenheit von Sauerstoff durchgeführt wird, wobei unter Abwesenheit von Sauerstoff ein Sauerstoffvolumenanteil kleiner als 0,1 %, insbesondere kleiner 0,01 und ganz besonders kleiner 0,001 %, bezogen auf das Gesamtvolumen des jeweilig benutzten Apparateteils (Kessel, Destillationskolonne) verstanden wird.

Die verwendeten Apparate sind vorzugsweise aus Edelstählen gefertigt, ebenso alle Rohrleitung und Kolonneneinbauten.

Das Verfahren kann diskontinuierlich oder kontinuierlich durchgeführt werden.

Bei der diskontinuierlichen Durchführung kann die basische Verbindung zu einer vorgelegten Menge an zu reinigendem N-Alkyl-pyrrolidon gegeben und die erhaltene Mischung absatzweise destilliert werden.

Vorzugsweise wird das Verfahren kontinuierlich durchgeführt.

Dazu werden die Ausgangsstoffe, das heißt die basische Verbindung und das zu reinigende N-Alkyl-pyrrolidon, kontinuierlich zusammengeführt. Sie können zum Beispiel kontinuierlich in ein Vorratsgefäß geführt und dort gemischt werden; aus diesem Vorratsgefäß kann die Destillation kontinuierlich gespeist werden.

Die basische Verbindung und das zu reinigende N-Alkyl-pyrrolidon können als separate Ströme direkt und kontinuierlich der Destillation zugeführt werden.

Das zu reinigende N-Alkyl-pyrrolidon wird vorzugsweise im Abtriebsteil der Kolonne zugeführt. Die basische Verbindung kann in Reinsubstanz oder als Lösung auf einem beliebigen Boden der Destillationskolonne zugeführt werden.

Die Destillation kann ein- oder mehrstufig, das heißt in einer oder mehreren Kolonnen erfolgen.

Für eine einstufige, kontinuierliche Destillation ist beispielsweise auch eine Trennwandkolonne geeignet.

In einer bevorzugten Ausführungsform wird eine kontinuierliche, zweistufige Destillation durchgeführt.

Dazu können das zu reinigende N-Alkylpyrrolidon (vorzugsweise zu reinigendes NMP) und die basische Verbindung (vorzugsweise NaOH) im Gemisch oder getrennt einer ersten Destillationskolonne zugeführt werden. In der ersten Destillationskolonnen werden vorzugsweise Leichtsieder wie Methylamin, insbesondere Wasser, bei einer Sumpftemperatur von 150 bis 250°C, insbesondere 180 bis 250°C, und einem Druck von 0,1 bis 5 bar, insbesondere 0,3 bis 3 bar über Kopf abgetrennt. In der zweiten Destillationskolonne kann dann die Reindestillation des Sumpfes der ersten Destillationskolonne unter vermindertem Druck erfolgen, zum Beispiel bei einer Sumpftemperatur von 80 bis 180°C, insbesondere 100 bis 160°C und einem Druck von 0,005 bis 0,5 bar, insbesondere 0,01 bis 0,3 bar. Das gereinigte N-Alkylpyrrolidon kann als Seitenabzug der zweiten Kolonne abgetrennt und der Sumpf, welcher die Verunreinigungen und die basische Verbindung, bzw. deren Umsetzungsprodukte, enthält, kontinuierlich ausgeschleust werden.

Eine besondere Ausführungsform des Verfahrens ist, die Destillation des zu reinigenden N-Alkyl-pyrrolidon im Gemisch mit zusätzlichem N-Alkyl-pyrrolidon aus dem Herstellungsprozess durchzuführen.

N-Alkyl-pyrrolidon, insbesondere NMP, kann durch Umsetzung von gamma-Butyrolacton mit dem entsprechenden Amin (Methylamin im Falle des NMP) erhalten werden. Bei dieser Umsetzung wird ein Produktstrom aus N-Alkyl-pyrrolidon (NMP), Wasser, nicht umgesetzten Ausgangstoffen (gamma-Butyrolacton und Methylamin) und gegebenenfalls Nebenprodukten erhalten. Dieser Produktstrom wird durch Destillation aufgearbeitet. Das zu reinigende N-Alkyl-pyrrolidon kann mit diesem Produktstrom in beliebigen Mengen gemischt werden. Das zu reinigende N-Alkyl-pyrrolidon kann die basische Verbindung bereits vor der Zusammenführung mit dem Produktstrom enthalten. Alternativ ist es natürlich auch möglich, die basische Verbindung dem Produktstrom zuzusetzen, so wird die basische Verbindung dem zu reinigenden N-Alkyl-pyrrolidon erst durch Mischen mit dem Produktstrom zugeführt.

Nach der Destillation enthält das gereinigte N-Alkylpyrrolidon auf 100 Gewichtsteile N-Alkyl-pyrrolidon
- insgesamt weniger als 0,5 Gewichtsteile der Verunreinigungen III - XII
- weniger als 0,05 Gewichtsteil Wasser und
- insgesamt weniger als 0,01 Gewichtsteil Verbindungen der Formel I und II

Insbesondere enthält das gereinigte N-Alkylpyrrolidon auf 100 Gewichtsteile N-Alkylpyrrolidon
- insgesamt weniger als 0,1 Gewichtsteile der Verunreinigungen III - XII
- weniger als 0,03 Gewichtsteile Wasser und
- insgesamt weniger als 0,0005 Gewichtsteile Verbindungen der Formel I und II

Das gereinigte N-Alkyl-pyrrolidon enthält insbesondere weniger als 5 Gew. %, bevorzugt weniger als 1 Gew. %, besonders bevorzugt weniger als 0,1 Gew. % der ursprünglich im verunreinigten N-Alkyl-pyrrolidon enthaltenen Gesamtmenge der Verbindungen der Formel I und II.

Das erhaltene, gereinigte N-Alkyl-pyrrolidon eignet sich aufgrund seiner hohen Reinheit erneut für die Herstellung von Elektroden für Lithium-Akkumulatoren.

### Beispiele

Zur Verdeutlichung der Erfindung sollen die nachfolgenden Beispiele dienen. Die in den Beispielen angegebenen Gehalte an Verunreinigungen sind durch Gaschromatographie (GC-Gerät HP6890, FID-Dektor, Stickstoff Trägergas mit 1,0 mL/min (const. flow); Split Ratio 1:50; Säule RTX-1, 30 m, 0,32 mm, 1,0 µm Film; Temperaturprogramm Start bei 80°C, dann 5°C/min bis 140°C, dann 5°C/min bis 200°C und 10 min isotherm, dann 10°C/min bis 340°C und 8 min isotherm) bestimmt. Wasserwerte wurden per Karl-Fischer-Titration bestimmt. Eingesetzt wurden NMP sowie die entsprechenden Verunreinigungen wie in den Beispielen angegeben. Alle Arbeitsschritte erfolgten in gereinigten Apparaten unter Stickstoffatmosphäre, die Abwesenheit von Fremdverbindungen wurde vor Versuchsbeginn per GC kontrolliert.

### Vergleichsbeispiel 1

Ein verunreinigtes NMP mit 0,5% Wasser und 0,031% Verunreinigungen der Formeln I - XII (Verunreinigung der Formel I und II, 0,004%; Verunreinigung der Formel III, 0,017%; Verunreinigung der Formeln IV - VI, 0,003%; Verunreinigung der Formeln VII - IX, 0,002%; Verunreinigung der Formeln X - XII, 0,005%) wurde mit 0,1 % NaOH (als reine NaOH berechnet, eingesetzt als 30%ige wässrige Lösung) versetzt und in einen Tankcontainer gepumpt Nach einer Verweilzeit von 4 h bei einer Temperatur von 15°C fielen ca. 100 kg eines Niederschlags bei einer Startmenge von 23,5 t NMP aus. Für die folgende zweistufige Destillation wurde der Tankinhalt über ein Steigrohr kontinuierlich mit einer Menge von 500 L/h in die erste Ventilbodenkolonne (40 Böden, Zulauf auf Boden 21) gefördert und der Zulauf nach Eintanken von 23,3 t gestoppt, so dass der Festostoff im Tankcontainer verblieb. Die Destillation erfolgte bei Sumpftemperaturen zwischen 180 und 185°C, einem Kolonnenkopfdruck von 530 mbar und einem Rücklauf- zu Abnahme-Verhältnis von etwa 1 zu 1. Der Kolonnensumpf wurde in der zweiten Ventilbodenkolonne (40 Böden, Zulauf auf Boden 21) geleitet und bei Sumpftemperaturen zwischen 165 und 170°C, einem Kolonnenkopfdruck von 220 mbar destilliert, das Produkt wurde über Kopf abgenommen. Der Natriumgehalt im Zulauf für die zweite Ventilbodenkolonne lag zwischen 0,0005 und 0,001 %,das Destillat wies weiterhin die Verunreinigungen der Formel I und II von 0,002 % und Verunreinigung der Formel IV - VI von 0,001 % auf und war frei von der Verunreinigung der Formel III, die Destillationsausbeute lag bei 90% und der Wassergehalt im Produkt betrug 0,005 %.

### Vergleichsbeispiel 2

Ein verunreinigtes NMP mit 1,0% Wasser und 0,074% Verunreinigungen der Formeln I - XII (Verunreinigung der Formel I und II, 0,003%; Verunreinigung der Formel III, 0,035%; Verunreinigung der Formeln IV - VI, 0,002%; Verunreinigung der Formeln VII - IX, 0,032%; Verunreinigung der Formeln X - XII, 0,002%) wurde mit 0,1 % NaOH (als reine NaOH berechnet, eingesetzt als 30%ige wässrige Lösung) versetzt und in einen Behälter gepumpt. Innerhalb einer Verweilzeit von 6 h bei einer Temperatur von 25 °C fiel ca. 0,1 kg eines Niederschlags bei einer Startmenge von 30 kg NMP aus. Für die folgende Destillation wurde der Inhalt des Behälters in die Blase einer Destillationsapparatur transferiert und absatzweise fraktioniert. Der Feststoff verblieb im Behälter. Die Destillation in einer Kolonne (Sulzer CY Packung, 13 Schüsse je 0,63 m, 1 Schuss je 0,30 m) mit Rücklaufteiler bei Sumpftemperaturen zwischen 130 bis 140°C und Drücken zwischen zu Beginn 1000 mbar (Leichtsiederabtrennung) und 90 mbar Kopfdruck bei einem Rücklauf- zu Abnahme-Verhältnis von 50 zu 1 destilliert. Es wurden insgesamt 30 Destillatfraktionen genommen. In den ersten fünf Fraktionen, die bei Kopftemperaturen von 55 - 118°C erhalten wurden, fand sich überwiegend Wasser (> 98%). Bei 100 mbar und einer Kopftemperatur von 122 - 126°C wurden die nachfolgenden Fraktionen 6 - 24 (zusammen ca. 21 kg) erhalten. Diese enthielten Wasser unter 0,1 %, NMP in Reinheiten über 99,8% allerdings noch die bereits in der Vorlage vorhandenen Verunreinigungen der Formeln I und II, 0,001 % und Formeln IV - VI, 0,001 %. Ab Fraktion 25 (zusammen ca. 4 kg) konnten per GC-Analytik die unerwünschten Verunreinigungen der Formeln I - XII nicht mehr detektiert werden, die Destillationsausbeute lag bei 13% und der Wassergehalt im Produkt betrug 0,005%.

### Erfindungsgemäße Beispiele

### Beispiel 1

Das Einsatzgemisch aus Vergleichsbeispiel 1 wurde über einen statischen Mischer mit einer Verweilzeit von 30 Sekunden bei einer Zulaufgeschwindigkeit des verunreinigten NMP von 500 Liter (L)/Stunde (h) und einer Temperatur von 15°C kontinuierlich mit 0,1% NaOH (als reine NaOH berechnet, eingesetzt als 30%ige wässrige Lösung) versetzt. Dieser Zulauf wurde im Gegensatz zu Vergleichsbeispiel 1 direkt in die erste Ventilbodenkolonne geleitet (Zulauf auf Boden 21) und dort innerhalb von 2 Minuten auf über 150°C erhitzt. Die Bildung eines Feststoffes wurde nicht beobachtet. Die Destillation erfolgte zweistufig analog Vergleichsbeispiel 1. Der Natriumgehalt im Zulauf für die zweite Ventilbodenkolonne lag zwischen 0,05 und 0,06%, die Destillationsausbeute lag bei 90%, per GC-Analytik konnten die Verunreinigungen der Formeln I - XII im Produkt nicht detektiert werden und der Wassergehalt betrug 0,005%.

### Beispiel 2

Das Einsatzgemisch aus Vergleichsbeispiel 2 wurde mit 0,1% NaOH (als reine NaOH berechnet, eingesetzt als 30%ige wässrige Lösung) über einen statischen Mischer mit einer Verweilzeit von 30 Sekunden durchmischt und über einen Vorheizer innerhalb von 10 Minuten auf 95°C erhitzt, wobei die Bildung eines Feststoffes nicht beobachtet wurde. Mit einer Zulaufgeschwindigkeit von 5 L/min wurde das Gemisch in die Blase einer Destillationsapparatur transferiert und direkt absatzweise analog Vergleichsbeispiel 2 fraktioniert (Aufheizen auf 130°C innerhalb von weiteren 20 Minuten). Es wurden insgesamt 20 Destillatfraktionen genommen. In den ersten fünf Fraktionen, die bei Kopftemperaturen von 55 - 118°C erhalten wurden, fand sich überwiegend Wasser (> 98%). Bei 100 mbar und einer Kopftemperatur von 122 - 126°C wurden die nachfolgenden Fraktionen 6 - 20 (zusammen ca. 25 kg) erhalten. Diese enthielten Wasser unter 0,1 %, NMP in Reinheiten über 99,8% und per GC-Analytik konnten die unerwünschten Verunreinigungen der Formeln I - XII nicht mehr detektiert werden. Die Destillationsausbeute lag bei 83% und der Wassergehalt im Produkt betrug 0,005%.

### Beispiel 3

Das Einsatzgemisch aus Vergleichsbeispiel 2 wurde mit 0,15% Kaliummethylat (kurz KOMe, als reine KOMe berechnet, eingesetzt als 25%ige methanolische Lösung) über einen statischen Mischer mit einer Verweilzeit von 30 Sekunden durchmischt und über einen druckstabilen Vorheizer innerhalb von 10 Minuten auf 95°C erhitzt, wobei die Bildung eines Feststoffes nicht beobachtet wurde. Mit einer Zulaufgeschwindigkeit von 10 L/min wurde das Gemisch in die Blase einer Destillationsapparatur transferiert und direkt absatzweise analog Vergleichsbeispiel 2 fraktioniert (Aufheizen auf 130°C innerhalb von weiteren 20 Minuten). Es wurden insgesamt 20 Destillatfraktionen genommen. In den ersten fünf Fraktionen, die bei Kopftemperaturen von 55 - 118°C erhalten wurden, fand sich überwiegend Wasser (> 98%) und ein kleiner Anteil an Methanol. Bei 100 mbar und einer Kopftemperatur von 122 - 126°C wurden die nachfolgenden Fraktionen 6 - 20 (zusammen ca. 25 kg) erhalten. Diese enthielten Wasser unter 0,1 %, NMP in Reinheiten über 99,8% und per GC-Analytik konnten die unerwünschten Verunreinigungen der Formeln I - XII und Methanol nicht mehr detektiert werden.

### Beispiel 4

Das Einsatzgemisch (500 Milliliter) aus Vergleichsbeispiel 1 wurde in einem Stahlautoklav mit 1 % Monomethylamin (als reines Amin berechnet, eingesetzt als 40%ige wässrige Lösung) innerhalb von 15 Minuten auf 100°C erwärmt und dann weiter auf 200°C für 6 h erhitzt, wobei sich ein Druck von ca. 20 bar aufbaute. Der feststofffreie Reaktionsaustrag wurde anschließend analog der Bedingungen des Vergleichsbeispiels 2 in einer Laborapparatur (Standarddestillationsaufbau mit Vigreux-Kolonne) destilliert. Es wurden ca. 90% NMP in der gewünschten Reinheit erhalten (Gehalt Monomethylamin < 0,002%), wobei der überwiegende Rest nur durch zu hohe Wassergehalte (> 0,1 %) verunreinigt war.

## Patentansprüche

1. Verfahren zur Reinigung von N-Alkyl-pyrrolidonen, welche durch eine bereits erfolgte Verwendung mindestens eine der Verunreinigungen der Formel I oder II 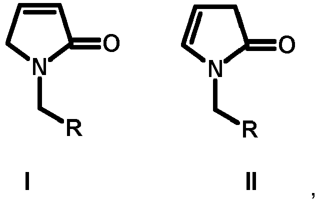 worin R für Wasserstoff oder eine C1- bis C20-Alkylgruppe steht, enthalten,
**dadurch gekennzeichnet, dass**
- dem zu reinigenden N-Alkyl-pyrrolidon eine basische Verbindung zugesetzt wird und die Temperatur des Gemisches spätestens 20 Minuten nach Zusatz der basischen Verbindung mindestens 80°C beträgt und
- N-Alkyl-pyrrolidon aus dem erhaltenen Gemisch abdestilliert wird.

2. Verfahren gemäß Anspruch 1, **dadurch gekennzeichnet, dass** es sich bei dem N-Alkyl-pyrrolidon um N-Methyl-pyrrolidon handelt.

3. Verfahren gemäß Anspruch 1 oder 2, **dadurch gekennzeichnet, dass** es sich bei der basischen Verbindung um eine Verbindung handelt, welche in einer Menge von 5 Gewichtsteilen in 100 Gewichtsteilen Wasser (bei 20°C, 1 bar) einen pH-Wert von mindestens 8 bewirkt.

4. Verfahren gemäß einem der Ansprüche 1 bis 3, **dadurch gekennzeichnet, dass** es sich bei der basischen Verbindung um Alkali- oder Erdalkalihydroxide handelt.

5. Verfahren gemäß einem der Ansprüche 1 bis 4, **dadurch gekennzeichnet, dass** die basische Verbindung in Mengen von 0,01 bis 10 Gewichtsteilen bezogen auf 100 Gewichtsteile N-Alkyl-pyrrolidon zugegeben wird.

6. Verfahren gemäß einem der Ansprüche 1 bis 5, **dadurch gekennzeichnet, dass** die Temperatur des Gemisches spätestens 5 Minuten nach Zusatz der basischen Verbindung mindestens 100°C beträgt.

7. Verfahren nach einem der Ansprüche 1 bis 6, **dadurch gekennzeichnet, dass** die basisch wirkende Verbindung während der Destillation zugesetzt wird.

8. Verfahren gemäß einem der Ansprüche 1 bis 7, **dadurch gekennzeichnet, dass** die Destillation bei einer Temperatur von 100 bis 300°C und einem Druck von 0,005 bis 10 bar durchgeführt wird.

9. Verfahren gemäß einem der Ansprüche 1 bis 8, **dadurch gekennzeichnet, dass** die Destillation in Abwesenheit von Sauerstoff durchgeführt wird.

10. Verfahren gemäß einem der Ansprüche 1 bis 9, **dadurch gekennzeichnet, dass** das Verfahren kontinuierlich durchgeführt wird.

11. Verfahren gemäß einem der Ansprüche 1 bis 10, **dadurch gekennzeichnet, dass** die Destillation des zu reinigenden N-Alkyl-pyrrolidon im Gemisch mit zusätzlichem N-Alkyl-pyrrolidon aus dem Herstellungsprozess erfolgt.

12. Verfahren gemäß einem der Ansprüche 1 bis 11, **dadurch gekennzeichnet, dass** das nach der Destillation erhaltene, gereinigte N - Alkyl-pyrrolidon, auf 100 Gewichtsteile N-Alkyl-pyrrolidon
- insgesamt weniger als 0,5 Gewichtsteile anderer Bestandteile
- weniger als 0,05 Gewichtsteile Wasser und
- insgesamt weniger als 0,005 Gewichtsteile Verbindungen der Formel I und II
enthält.

13. Verfahren zur Verwendung, Reinigung und Wiederverwendung von N-Alkyl-pyrrolidonen, **dadurch gekennzeichnet, dass**
a) ein Polymer in einem N-Alkyl-pyrrolidon gelöst wird,
b) Elektroden von Lithium-ionen-akkumulatoren mit diesem Polymer hergestellt werden und dabei ein verunreinigtes N-Alkyl-pyrrolidon zurückgewonnen wird, welches mindestens eine der Verunreinigungen der Formel I oder II 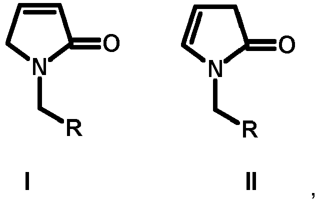 worin R für Wasserstoff oder eine C1- bis C20-Alkylgruppe steht, enthält,
c) dem zurückgewonnenen und zu reingenden N-Alkyl-pyrrolidon eine basische Verbindung zugesetzt wird und die Temperatur des Gemisches spätestens 20 Minuten nach Zusatz der basischen Verbindung auf mindestens 80°C erhöht wird,
d) das N-Alkyl-pyrrolidon aus dem erhaltenen Gemisch abdestilliert wird, und
e) erneut als Lösemittel für die Herstellung von Elektroden für Lithium-ionen-Akkumulatoren verwendet wird.

## Claims

1. A process for purifying N-alkylpyrrolidones which due to a previous use comprise at least one of the impurities of the formula I or II 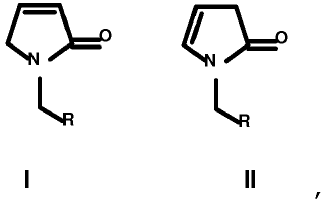 where R is hydrogen or a C1-C20-alkyl group,
wherein
- a basic compound is added to the N-alkylpyrrolidone to be purified and the temperature of the mixture is at least 80°C not more than 20 minutes after addition of the basic compound and
- N-alkylpyrrolidone is distilled off from the mixture obtained.

2. The process according to claim 1, wherein the N-alkylpyrrolidone is N-methylpyrrolidone.

3. The process according to claim 1 or 2, wherein the basic compound is a compound which in an amount of 5 parts by weight in 100 parts by weight of water (at 20°C, 1 bar) gives a pH of at least 8.

4. The process according to any of claims 1 to 3, wherein the basic compound is alkali metal hydroxides or alkaline earth metal hydroxides.

5. The process according to any of claims 1 to 4, wherein the basic compound is added in amounts of from 0.01 to 10 parts by weight per 100 parts by weight of N-alkylpyrrolidone.

6. The process according to any of claims 1 to 5, wherein the temperature of the mixture is at least 100°C not more than 5 minutes after addition of the basic compound.

7. The process according to any of claims 1 to 6, wherein the basic compound is added during the distillation.

8. The process according to any of claims 1 to 7, wherein the distillation is carried out at a temperature of from 100 to 300°C and a pressure of from 0.005 to 10 bar.

9. The process according to any of claims 1 to 8, wherein the distillation is carried out in the absence of oxygen.

10. The process according to any of claims 1 to 9, wherein the distillation is carried out continuously.

11. The process according to any of claims 1 to 10, wherein the distillation of the N-alkylpyrrolidone to be purified is carried out in admixture with additional N-alkylpyrrolidone from the production process.

12. The process according to any of claims 1 to 11, wherein the purified N-alkylpyrrolidone obtained after the distillation comprises, per 100 parts by weight of N-alkylpyrrolidone,
- a total of less than 0.5 part by weight of other constituents,
- less than 0.05 part by weight of water and
- a total of less than 0.005 part by weight of compounds of the formulae I and II.

13. A process for the use, purification and reuse of N-alkylpyrrolidones, wherein
a) a polymer is dissolved in an N-alkylpyrrolidone,
b) electrodes of lithium ion accumulators are produced using this polymer and a contaminated N-alkylpyrrolidone comprising at least one of the impurities of the formula I or II 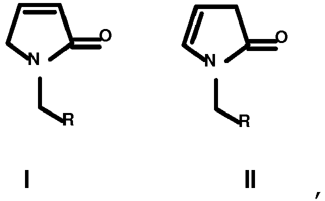 where R is hydrogen or a C1-C20-alkyl group, is recovered,
c) a basic compound is added to the recovered N-alkylpyrrolidone to be purified and the temperature of the mixture is increased to at least 80°C not more than 20 minutes after addition of the basic compound,
d) the N-alkylpyrrolidone is distilled off from the mixture obtained and
e) is reused as solvent for the production of electrodes for lithium ion accumulators.

## Revendications

1. Procédé pour la purification de N-alkylpyrrolidones, qui contiennent, par une utilisation qui a déjà eu lieu, au moins une des impuretés de formule I ou II 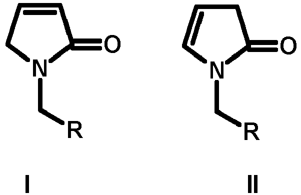 dans lesquelles R représente hydrogène ou un groupe C₁-C₂₀-alkyle, **caractérisé en ce que**
- la N-alkylpyrrolidone à purifier est additionnée d'un composé basique et la température du mélange, au plus tard 20 minutes après l'addition du composé basique, est d'au moins 80°C et
- la N-alkylpyrrolidone est éliminée par distillation du mélange obtenu.

2. Procédé selon la revendication 1, **caractérisé en ce qu'**il s'agit, pour la N-alkylpyrrolidone, de N-méthylpyrrolidone.

3. Procédé selon la revendication 1 ou 2, **caractérisé en ce qu'**il s'agit, pour le composé basique, d'un composé qui provoque, en une quantité de 5 parties en poids dans 100 parties en poids (à 20°C, 1 bar), un pH d'au moins 8.

4. Procédé selon l'une quelconque des revendications 1 à 3, **caractérisé en ce qu'**il s'agit, pour le composé basique, d'hydroxydes de métal alcalin ou de métal alcalino-terreux.

5. Procédé selon l'une quelconque des revendications 1 à 4, **caractérisé en ce que** le composé basique est ajouté en des quantités de 0,01 à 10 parties en poids, par rapport à 100 parties en poids de N-alkylpyrrolidone.

6. Procédé selon l'une quelconque des revendications 1 à 5, **caractérisé en ce que** la température du mélange, au plus tard 5 minutes après l'addition du composé basique, est d'au moins 100°C.

7. Procédé selon l'une quelconque des revendications 1 à 6, **caractérisé en ce que** le composé à effet basique est ajouté pendant la distillation.

8. Procédé selon l'une quelconque des revendications 1 à 7, **caractérisé en ce que** la distillation est réalisée à une température de 100 à 300°C et à une pression de 0,005 à 10 bars.

9. Procédé selon l'une quelconque des revendications 1 à 8, **caractérisé en ce que** la distillation est réalisée en l'absence d'oxygène.

10. Procédé selon l'une quelconque des revendications 1 à 9, **caractérisé en ce que** le procédé est réalisé en continu.

11. Procédé selon l'une quelconque des revendications 1 à 10, **caractérisé en ce que** la distillation de la N-alkylpyrrolidone à purifier a lieu en mélange avec de la N-alkylpyrrolidone supplémentaire provenant du procédé de préparation.

12. Procédé selon l'une quelconque des revendications 1 à 11, **caractérisé en ce que** la N-alkylpyrrolidone purifiée, obtenue après la distillation, contient, pour 100 parties en poids de N-alkylpyrrolidone,
- au total moins de 0,5 partie en poids d'autres constituants,
- moins de 0,05 partie en poids d'eau et
- au total moins de 0,005 partie en poids de composés de formule I et II.

13. Procédé pour l'utilisation, la purification et la récupération de N-alkylpyrrolidones, **caractérisé en ce que**
a) un polymère est dissous dans une N-alkylpyrrolidone,
b) des électrodes d'accumulateurs lithium-ion sont fabriquées à l'aide de ce polymère et une N-alkylpyrrolidone contaminée est récupérée, qui contient au moins une des impuretés de formule I ou II 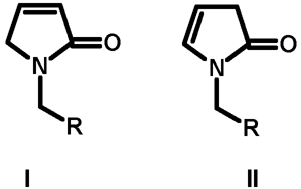 dans lesquelles R représente hydrogène ou un groupe C₁-C₂₀-alkyle,
c) la N-alkylpyrrolidone récupérée et à purifier est additionnée d'un composé basique et la température du mélange, au plus tard 20 minutes après l'addition du composé basique, est augmentée à au moins 80°C et
d) la N-alkylpyrrolidone est éliminée par distillation du mélange obtenu et
e) de nouveau utilisée comme solvant pour la fabrication d'électrodes pour des accumulateurs lithium-ion.
